Europäisches Patentamt

European Patent Office

Office européen des brevets

(11) Publication number: **0 315 786**
A1

# EUROPEAN PATENT APPLICATION

(21) Application number: 88116999.9

(51) Int. Cl.4: **C12P 13/04 , C12N 9/10**

(22) Date of filing: 13.10.88

(30) Priority: 14.10.87 NL 8702449

(43) Date of publication of application:
17.05.89 Bulletin 89/20

(84) Designated Contracting States:
AT BE CH DE ES FR GB GR IT LI NL SE

(71) Applicant: STAMICARBON B.V.
Mijnweg 1
NL-6167 AC Geleen(NL)

(72) Inventor: Van den Tweel, Will J.J.
Thorbeckestraat 152
NL-6702 BW Wageningen(NL)
Inventor: Ogg, Ron L.H.P.
Azaleastraat 32
NL-6163 CD Geleen(NL)
Inventor: De Bont, Jan A.M.
Lawickse allee 68
NL-6707 AK Wageningen(NL)

(54) **Process for the preparation of a d-alfa-amino acid from the corresponding alfa-keto acid.**

(57) The invention relates to a process for the preparation of a D-α-amino acid from the corresponding α-keto acid and an amino donor with the aid of a D-transaminase, in which p-hydroxyphenylglyoxylate is contacted with a preparation of Pseudomonas putida NCIB 12565, or a mutant thereof, containing D-transaminase, in the presence of a molar excess of amino donor with respect to the p-hydroxyphenylglyoxylate, which results in optically pure D-p-hydroxyphenylglycine.

EP 0 315 786 A1

## PROCESS FOR THE PREPARATION OF A D-α-AMINO ACID FROM THE CORRESPONDING α-KETO ACID

The invention relates to a process for the preparation of a D-α-amino acid from the corresponding α-keto acid and an amino donor with the aid of a D-transaminase. In particular, the process relates to the preparation of D-p-hydroxyphenylglycine. This substance is of particular importance as an intermediate for compounds with pharmaceutical properties, particularly in the preparation of semisynthetic penicillins and cephalosporins, for example in the preparation of antibiotics such as amoxycillin.

In the chemical preparation of p-hydroxyphenylglycine a racemic mixture of DL-p-hydroxyphenylglycine is obtained from which, via a laborious purification, the D-configuration that is active in the desired applications must then be separated. L-p-hydroxyphenylglycine shows no activity in the desired pharmaceutical preparations. There is therefore a need for a simple process for the preparation of D-p-hydroxyphenylglycine in optically pure form.

Recent developments in the field of D-transaminases, as described in DE-A-3447023, have shown that various α-keto acids (or salts thereof) can be converted into the corresponding D-α-amino acids with the aid of an amino donor and a D-transaminase obtained from Bacillus licheniformis ATCC 9945.

The reaction referred to is an equilibrium reaction and can therefore also be effected in the opposite direction, in which a D-α-amino acid is converted into the corresponding α-keto acid with the aid of an amino acceptor. DE-A-3447023 describes the conversion of cephalosporin C (CPC) into 7-aminocephalosporanic acid (7-ACA) and mentions the amino acids: D-alanine, D-α-aminoadipic acid, D-aspartic acid, D- glutamic acid, D-leucine, D-lysine, D-methionine, D-phenylalanine, D-proline, D-serine, D-tryptophane, D-tyrosine, D-valine, D-cystine and D-alanine-D-alanine. D-p-Hyroxyphenylglycine is not mentioned, which, as demonstrated in the experimental part of the present application, is due to the fact that the D-transaminase obtained from Bacillus licheniformis has no activity for the conversion of D-p-hydroxyphenylglyoxylate into D-p-hydroxyphenylglycine. DE-A-3447023 does not either in fact give a decisive answer about the optical purity of the D-α-amino acids to be obtained, and the forming activity is only very low (see comparative example B of the present application).

It should be noted that other enzymatic routes have been described for the preparation of D-α-amino acids. Examples are the processes in which hydantoins are converted into the corresponding D-α-amino acid partly with the aid of a hydantoinase. A disadvantage of such a process in the preparation of D-p-hydroxyphenylglycine is that the required hydantoin starting material (DL-p-hydroxyphenylhydantoin) is relatively expensive and difficult to obtain, whereas the starting material for the transamination reaction according to the invention (p-hydroxyphenylglyoxylate) can be obtained in a simple reaction step from relatively cheap and sufficiently available p-hydroxymandelic acid. The amino donor (preferably L-glutamate) required in the process according to the invention is also readily available at a low price.

The aim of the invention, now, is to provide a process for the preparation of optically pure D-p-hydroxyphenylglycine by converting p-hydroxyphenylglyoxylate into D-p-hydroxyphenylglycine in a simple manner with the aid of a suitable D-transaminase and an amino donor.

Surprisingly, it has now been found that a microorganism which was isolated in an investigation into the microbial breakdown of D- and L-phenylglycine (see Arch. Microbiol, 1986, 144 pp. 169-174) and classified as a Pseudomonas putida and which appeared capable of transaminating both steroisomers of phenylglycine, has a highly specific D-transaminase activity for the preparation of D-p-hydroxyphenylglycine from p-hydroxyphenylglyoxylate. This microorganism is filed with the National Collection of Industrial Bacteria at Aberdeen as NCIB 12565.

Pseudomonas putida NCIB 12565 is a mobile, Gram negative, rod-shaped bacterium with several polar flagellae. The organism is oxidase-positive and has arginine dihydrolase, urease and β-galactosidase activity. Pseudomonas putida NCIB 12565 forms a fluorescent pigment, is strictly aerobic, does not grow at 41□C, is not capable of liquefying gelatin and shows no signs of growth under denitrifying conditions.

The process according to the invention for the preparation of a D-α-amino acid from the corresponding α-keto acid and an amino donor with the aid of a D-transaminase is characterized in that p-hydroxyphenylglyoxylate is contacted with a preparation of Pseudomonas putida NCIB 12565, or a mutant thereof, containing D-transaminase, in the presence of a molar excess of amino donor with respect to the p-hydroxyphenylglyoxylate, which results in optically pure D-p-hydroxyphenylglycine. This results in a simple preparation method for D-p-hydroxyphenylglycine with a high degree of optical purity from p-hydroxyphenylglyoxylate that can be obtained at a low cost from relatively cheap and sufficiently available p-hydroxy-mandelic acid.

Arch. of Microbiology 1986, 144 p. 170 describes the isolation and characterization of the Pseudomonas putida concerned. It also mentions that the strain can grow on different substrates, including D- and L-phenylglycine and D-p-hydroxyphenylglycine, as the only C-and energy-source, but that no growth occurs on trehalose, DL-o-hydroxyphenylglycine, L-p-hydroxyphenylglycine or o-hydroxybenzoate as substrate. Because, besides a transamination, various other enantioselective reactions can take place as initial step in the metabolism of D-p-hydroxyphenylglycine, similarly as in the metabolism of phenylalanine, such as decarboxylation (see Boeker & Snell (1972) in 'The Enzymes' Vol. 6, pp. 217-253, Academic Press), oxidative decarboxylation (see Koyama (1982), J. Biochem 92 p. 1235-1240) or hydroxylation (see Guroff & Ito (1964), J. Bio.Chem. 240, p. 1175-1184), the last three of which are, unlike the transamination, irreversible reac tions, the stereoselective effect in the transamination according to the invention cannot be deduced from the aforementioned article in Arch. of Microbiol.

A different publication (J. Gen. Microbiol. 1987, 133 p. 745-754) on the metabolism of D- and L-phenylglycine in a Flavobacterium species does not contain any indications referring to the initial step in the breakdown of p-hydroxyphenylglycine either.

In order to obtain sufficient D-transaminase, Pseudomonas putida NCIB 12565 was cultured in a 30 l fermenter with the aid of a mineral salts medium (composition per litre of demineralized water: 2.0 g of ammonium chloride, 0.1 g of ammonium sulphate, 0.85 g of monosodium phosphate, 1.55 g of dipotassium phosphate, 0.075 g of magnesium chloride, 0.1 g of yeast extract, 0.2 g of sodium chloride and a trace element solution containing Zn, Ca, Fe, Mo, Cu, Co and Mn), to which 5.0 g of D-p-hydroxyphenylglycine had been added (end pH: 7.0).

The fermentation temperature was 30¤C, the speed of the stirrer 300 rpm and air was supplied at a rate of 2 l per minute. After sterilization with steam (45 minutes at 120¤C), the fermenter was inoculated with 0.5 l of a culture of Pseudomonas putida NCIB 12565. 21 hours after inoculation, the cultured cells were harvested by centrifugation.
The optimum incubation temperature is between 25 and 35¤C. A neutral medium (pH.7.0) is not necessary, but is desirable for optimum incubation. In general, a pH value of between 5.5 and 9 is very suitable. Under optimum conditions the generation time of Pseudomonas putida NCIB 12565 on D-p-hydroxzphenylglycine is 1.5 hours.

Where there is mention of preparations of Pseudomonas putida NCIB 12565 in this patent application, this is understood to mean whole cells, cells-free extracts or purified enzyme preparations obtained therefrom, which may or may not have been immobilized. The invention also includes mutants derived from Pseudomonas putida NCIB 12565 with D-transaminase activity.

In the process according to the invention p-hydroxyphenylglyoxylate is contacted with a preparation of Pseudomonas putida NCIB 12565 containing a D-transaminase under conditions which will be dealt with in the following.

It is preferable to ensure that under production conditions the preparation contains very little or no p-hxdroxyphenylglyoxylate decarboxylase, because this results in an undesired decarboxylation of the substrate into p-hydroxybenzaldehyde. This can be effected in different manners, e.g. by using a cell-free extract from which the p-hydroxyphenylglyoxylate decarboxylase has been removed, by choosing production conditions (pH, temperature, etc.) at which the decarboxylase has virtually no activity with respect to the transaminase, or by using a mutant of Pseudomonas putida NCIB 12565 without an active p-hydroxyphenylglyoxylate decarboxylase.

The applicant removed this p-hydroxyphenylglyoxalate decarboxylase as follows: cells of Pseudomonas putida NCIB 12565 were broken open at 4¤C by ultrasonic vibration. Then the cell walls and cell membranes were removed by centrifugation. The clear supernatant contained D-p-hydroxyphenylglycine transaminase that is capable of converting p-hydroxyphenylglyoxylate into D-p-hydroxyphenylglycine. This supernatant also contained an enzyme that is capable of converting p-hydroxyphenylglyoxylate into p-hydroxybenzaldehyde, namely p-hydroxyphenylglyoxylate decarboxylase. This enzyme was partly removed by ammonium sulphate precipitation. Ammonium sulphate was added to the supernatant to 30% saturation, after which the precipitate was removed by centrifugation and the resulting supernatant was subsequently saturated with ammonium sulphate to 35%. The precipitated protein was removed by centrifugation and then resuspended in a 50 mM potassium phosphate buffer with a pH of 7.0. The protein fraction thus obtained had a high D-p-hydroxyphenylglycine transaminase activity in relation to the p-hydroxyphenylglyoxylate decarboxylase activity.

A preparation containing D-transaminase that is absolutely free from p-hydroxyphenylglyoxylate decarboxylase activity can be obtained via the following steps:
    1) ammonium sulphate precipitation
    2) ion exchange chromatography on a DEAE column
    3) gel permeation on a Sephacryl-300 column

4) ion exchange chromatography on a mono-Q column by means of Fast Protein Liquid Chromatography (FPLC).

The conversion of p-hydroxyphenylglyoxylate into D-p-hydroxyphenylglycine with the aid of the D-transaminase according to the invention proceeds excellently in an aqueous medium, but this reaction can also take place in the presence of organic media such as dibutyl phthalate, hexadecane and perfluorine hydrocarbons. How the reaction is to take place can easily be determined by a person skilled in the art.

Where this patent application mentions p-hydroxyphenylglyoxylate this is understood to be p-hydroxyphenylglyoxylic acid or salts thereof such as K-, Na- and ammonium salts or salts with quaternary ammonium compounds and organic amines.

The conversion proceeds excellently at temperatures between 5 and 50¤C. Good results have been obtained at about 30¤C. There are no stringent requirements regarding the pH during conversion. Good results are obtained at a pH between 5 and 11; usually the reaction will be effected at a pH between 7.5 and 9.5.

Since the transamination reaction implies the transfer of an amino group, the reaction medium shall contain an amino donor, which, because the reaction is an equilibrium reaction, shall be present in a molar excess with respect to the p-hydroxyphenylglyoxylate in order to ensure that the reaction results in a good yield of the desired D-p-hydroxyphenylglycine product. Various compounds, such as L-glutamate and L-phenylglycine, may be used as amino donor, but from the viewpoint of process economy it is preferable to use the cheapest amino donor, L-glutamate.

p-Hydroxyphenylglyoxylate is preferably present in an amount of at least 0.5 mmol per litre at the beginning of the reaction. Depending on which of the possible preparations of Pseudomonas putida NCIB 12565 is used, it is more or less recommendable to add a catalytic amount of pyridoxal phosphate that is also required as factor in the conversion. When whole cells are used, the amount of pyridoxal phosphate contained in the cells is sufficient. When cell-free preparations etc. are used, between 1 and 100 micromoles of pyridoxal phosphate per litre is preferably added.

As far as process technology is concerned, the aim is to obtain maximum volumetric productivity at the highest possible product concentration. Reasonable results have so far been obtained with protein concentrations from 0.4 grams of protein per litre.

After the enzyme-containing preparation has been removed from the reaction mixture, the p-hydroxyphenylglycine can be obtained in a known manner with methods such as centrifugation, evaporation, crystallization and/or separation in an ion exchanger. A suitable method is to acidify the reaction mixture to dissolve all p-hydroxyphenylglycine, then remove the protein and set the pH of the filtrate to 4-7 so that the p-hydroxyphenylglycine precipitates and can be removed.

The invention is further elucidated with the following examples, without, however, being limited thereto.

Examples

The manner in which Pseudomonas putida NCIB 12565 was isolated and cultured in a 30 l dish and the manner in which p-hydroxyphenylglyoxylate decarboxylase can be removed from preparations thereof or in which these preparations can be completely purified has already been indicated in the introduction.

In the framework of the investigation the preparation reaction of p-hydroxyphenylglycine from p-hydroxyphenylglyoxylate as well as the reverse reaction were studied.

A spectrophotometric method was used to follow the conversion in the transamination of D-p-hydroxyphenylglycine. The transaminase activity can be determined spectrophotometrically (detection limit about 0.5 nmoles per minute per mg of protein) by making use of the fact that p-hydroxyphenylglyoxylate and p-hydroxybenzaldehyde both have a molar absorption coefficient of 4500 $M^{-1}$ $cm^{-1}$ at 340 nm (pH 7.0), whereas D-p-hydroxyphenylglycine shows no light absorption under these conditions. For example, the following components were added to the reaction mixture (total volume 1 ml) in a cuvette: 50 micromoles of cell-free extract, 15 micromoles of α-ketoglutarate, 0.1 micromole of pyridoxal phosphate and 100 micromoles of potassium phosphate buffer with a pH of 7.0. Then the reaction was started by adding 10 micromoles of D-p-hydroxyphenylglycine and the increase in absorbance was determined at 340 nm with the aid of a recorder.

For the determination of the enantioselectivity of the reaction use was made of a determination method that has recently been described for the separation of D- and L-amino acids with the aid of HPLC (Buck & Krummen (1984) J. Chromatogr. 315: 279-285). This method appeared to be very suitable for determining the enantiomeric purity of D-p-hydroxyphenylglycine from which the enantiomeric excess can be derived. To this effect, samples containing p-hydroxyphenylglycine were derived with o-phthaldialdehyde-N-tert.butyloxycarbonyl-L-cystein and the diastereoisomers obtained were then separated on a $C_{18}$ column (100 x 3 mm). A mixture of methanol and 50 mM potassium phosphate buffer with a pH of 7.0 (1 : 1 (v/v)) was used as eluent and the flow was 0.5 ml min⁻¹. Detection took place with the aid of a fluorescensce detector (excitation 344 nm, emission 443 nm). Under these conditions the derived L-enantiomer presented a retention time of 5.8 min, while the derivative of the D-enantiomer of p-hydroxyphenylglycine had a retention time of 8.6 minutes.

Examples I through V: Pseudomonas putida NCIB 12565

Example I

2 mg of cell-free extract obtained via ammonium sulphate precipitation (as described on page 5, lines 13 through 29 of this application) was incubated with 5 ml of an aqueous solution of 60 mM of L-glutamate, 100 mM of potassium phosphate buffer with a pH of 7.0, 0.1 mM of pyridoxal phosphate and 0.9 mM of p-hydroxyphenylglyoxylate at 30¤C. This resulted in the formation of D-p-hydroxyphenylglycine. After 30 minutes an equilibrium had been reached and the concentration of p-hydroxyphenylglyoxylate had dropped to 0.07 mM, while 0.25 mM of D-p-hydroxyphenylglycine had been formed. In addition, 0.58 mmol of p-hydroxybenyaldehyde had been formed. With the aid of the HPLC method mentioned above it was demonstrated that no L-p-hydroxyphenylglycine had been formed in this incubation. Under the aforementioned conditions an initial forming activity was calculated of 21 nmol per minute per mg of protein.

Example II

2 mg of the cell-free extract as used in example I was incubated with 5 ml of an aqueous solution of 60 mM of L-glutamate, 100 mM of Tris/HCl buffer with a pH of 8.5, 0.1 mM of pyridoxal phosphate and 0.9 mM of p-hydroxyphenylglyoxylate at 30¤C.
After 10 minutes an equilibrium was reached and the composition of the reaction mixture was 0.25 mM of D-p-hydroxyphenylglycine, 0.40 mM of p-hydroxyphenylglyoxylate and 0.25 mM of p-hydroxybenzaldehyde. The forming activity of D-p-hydroxyphenylglycine under these conditions was initially 74 nmol per minute per mg of protein.

Example III

Example II was repeated, but now with a higher concentration of L-glutamate, namely 1200 mM of L-glutamate. All other conditions were the same as in example II. After 30 minutes (equilibrium) the composition of the reaction mixture was 0.76 mM of D-p-hydroxyphenylglycine, 0.05 mM of p-hydroxyphenylglyoxylate and 0.09 mM of p-hydroxybenzaldehyde. The forming activity of D-p-hydroxyphenylglycine was initially 455 nmol per minute per mg of protein. With the aid of HPLC, the enantiomeric excess of D-p-hydroxyphenylglycine was shown to be 98.2%.

Example IV

In a manner similar to that described in example I, but now with a partly purified enzyme preparation obtained by gel permeation (see page 5, lines 13 through 35), the same bioconversion was effected. When 1.5 mM of p-hydroxyphenylglyoxylate was used, the reaction mixture contained 0.87 mM of D-p-hydroxyphenylglycine, 0.48 mM of p-hydroxybenzaldehyde and 0.15 mM of p-hydroxyphenylglyoxylate after 14 hours' incubation (at 30¤C). An initial forming activity was found of 25 nmol per minute per mg of protein.

Example V

During incubation (30¤C) of an enzyme preparation (obtained according to the method described on page 5, line 13 through page 6, line 2) which had been freed from decarboxylase activity by means of ion chromatography on a mono-Q column (FPLC) with L-glutamate (50 mM), p-hydroxyphenylglyoxylate (1.0 mM) and pyridoxal phosphate (0.1 mM) in a potassium phosphate buffer (100 mM, pH 7.0), p-hydroxyphenylglyoxylate was transaminated to produce D-p-hydroxyphenylglycine. After one hour (equilibrium) the reaction mixture contained 0.21 mM of D-p-hydroxyphenylglycine and 0.79 mM of p-hydroxyphenylglyoxylate. Under these conditions the transaminase activity was initially 17 nmol per minute per mg of protein. With the aid of HPLC, the enantiomeric excess of D-p-hydroxyphenylglycine was shown to be more than 99%.

Comparative examples A and B: Bacillus licheniformis ATCC 9945

Example A

A sample of the Bacillus licheniformis ATCC 9945 containing D-transimanase as described in DE-A-3447023 (National Collections of Industrial and Marine Bacteria at Aberdeen (collection number 8062)) was cultured (30¤C) in a 5 l Erlenmeyer filled with 0.5 l of 'original culturing medium' containing 1% of yeast extract, 0.8% of nutrient broth, and 0.5% of DL-glutamic acid (pH 7.2) (according to DE-A-3447023). After culturing, the cells were harvested by centrifugation, washed once with potassium phosphate buffer (100 mM, pH 7.0), and resuspended in the same buffer. The cells thus obtained were then broken open with ultrasonic vibration. The resulting homogenate was centrifuged (15 min, 27000 g at 4¤C), after which part of the supernatant (protein concentration 25 mg of protein per ml) was used to demonstrate D-transaminase activity for D-p-hydroxyphenylglycine. In the spectrophotometric assay described in the introduction of the experimental part no D-p-hydroxyphenylglycine transaminase activity could be demonstrated at a pH of 7.0 or at a pH of 8.0. Even when 0.2% D-p-hydroxyphenylglycine was added to the 'original' culturing medium, no D-p-hydroxyphenylglycine transaminase activity was observed.

Example B

In order to be certain that the enzyme used in comparative example A had D-transaminase activity, it was also investigated whether D-alanine (one of the substrates from DE-A-3.447.023 for which the enzyme activity appears to be relatively high) is transaminated into pyruvate. To this effect two incubations were carried out: (1) cell-free extract and 5 mM of D-alanine in 50 mM of potassium phosphate buffer with a pH of 8.0 and (2) cell-free extract, 6 mM of α-ketoglutarate, 0.1 mM of pyridoxal phosphate and 5 mM of D-alanine in 100 mM of potassium phosphate with a pH of 8.0. Samples were taken at different intervals, which were then boiled for 10 minutes. 50 microlitres of these boiled samples was then added to a cuvette containing 0.5 unit of lactate dehydrogenase and 0.2 mM of NADH in 100 mM of potassium phosphate buffer with a pH of 7.0. If pyruvate had been formed during the incubation this would then be reduced to lactate, which would result in a reduction in absorbance at 340 nm. The experiments showed that no pyruvate had been formed in incubation mixture (1), whereas it had been formed in incubation mixture (2). The activity observed was initially 2.3 nmol per minute per mg of protein.

From these experiments it can be concluded that Bacillus licheniformis ATCC 9945 has no enzyme that is capable of transaminating D-p-hydroxyphenylglycine. Incubation experiments also showed that, unlike Pseudomonas putida NCIB 12565, Bacillus cannot grow on D-p-hydroxyphenylglycine as the only C- and energy-source.

Claims

1. Process for the preparation of a D-α-amino acid from the corresponding α-keto acid and an amino donor with the aid of a D-transaminase, characterized in that p-hydroxyphenylglyoxylate is contacted with a preparation of Pseudomonas putida NCIB 12565, or a mutant thereof, containing D-transaminase, in the presence of a molar excess of amino donor with respect to the p-hydroxyphenylglyoxylate, which results in the optically pure D-p-hydroxyphenylglycine.

2. Process for the preparation of optically pure D-p-hydroxyphenylglycine according to claim 1, characterized in that the preparation containing D-transaminase has no or only a negligible amount of decarboxylase activity for p-hydroxyphenylglyoxylate.

3. Process according to claim 1 or 2, characterized in that the conversion is effected in an aqueous medium.

4. Process according to any one of claims 1-3, characterized in that the conversion is effected at a temperature of 5-50¤C.

5. Process according to any one of claims 1-4, characterized in that the conversion is effected at a pH of 5-11.

6. Process according to claim 5, characterized in that the conversion is effected at a pH of 7.5-9.5.

7. Process according to any one of claims 1-6, characterized in that L-glutamate is used as amino donor.

8. Process according to any one of claims 1-7, characterized in that the conversion is effected with an initial concentration of p-hydroxyphenylglyoxylate of at least 0.5 mmol per litre.

9. Use of a preparation of Pseudomonas putida NCIB 12565 containing D-transaminase for transamination.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 152 275 (G.D. SEARLE & CO.) * Page 1, line 1 - page 2, line 9; page 6, lines 12-19; page 8, lines 13,14; page 10, lines 16-25; page 7, lines 21-22; page 12, line 20; claim 1 * | 1,4,5,6 ,7 | C 12 P 13/04 C 12 N 9/10 |
| X,D | ARCHIVES OF MICROBIOLOGY, vol. 144, 1986, pages 169-174, Springer-Verlag, Berlin, DE; W.J.J. VAN DEN TWEEL et al.: "Microbial metabolism of D- and L-phenylglycine by Pseudomonas putida LW-4" * Figure 1; page 172, column 2, line 24 - page 173, column 1, line 14; figure 4 * | 9 | |
| Y,D | IDEM | 1,4,5,6 ,7 | |
| Y | EP-A-0 186 035 (HOECHST AG) * Claims 1,6; page 2, lines 18-21 * & DE-A-3 447 023 (Cat. Y,D) | 1,4,5,6 ,7 | TECHNICAL FIELDS SEARCHED (Int. Cl.4) |
| P,X | BIOLOGICAL ABSTRACTS, vol. 86, abstract no. 112983, Philadelphia, PA, US; W.J.J. VAN DEN TWEEL et al.: "The involvement of an enantioselective transaminase in the metabolism of D-3 and D-4 hydroxyphenylglycine in Pseudomonas-putida LW-4", & APPL. MICROBIOL. BIOTECHNOL. 1988. vol. 29, no. 2-3, 224-230 * Abstract * | 1-9 | C 12 P C 12 N |

The present search report has been drawn up for all claims

| Place of search | Date of completion of the search | Examiner |
|---|---|---|
| THE HAGUE | 28-01-1989 | VAN PUTTEN A.J. |